# EUROPEAN PATENT SPECIFICATION

(11) **EP 4 313 173 B1**
(45) Date of publication and mention of the grant of the patent: **04.11.2026**
(21) Application number: 22781873.9
(22) Date of filing: 17.03.2022
(51) Int. Cl.: A61K 49/00, C07D 241/12, C07D 241/20

(54) **PURIFICATION OF SUBSTITUTED DIAMINOPYRAZINE DICARBOXYLIC ACIDS**
REINIGUNG VON SUBSTITUIERTEN DIAMINOPYRAZINDICARBONSÄUREN
PURIFICATION D'ACIDES DICARBOXYLIQUES DIAMINOPYRAZINES SUBSTITUÉS

(30) Priority: 31.03.2021 US 202163168518 P
(43) Date of publication of application: 07.02.2024
(73) Proprietor: MediBeacon Inc., St. Louis MI 63141 (US)
(72) Inventor: ROGERS, Thomas, St. Louis, Missouri 63141 (US); ADAMS, David, St. Louis, Missouri 63141 (US); HUA, Xiaogang, St. Louis, Missouri 63141 (US)
(74) Representative: Watermeyer, Nicholas
(86) International application number: PCT/US2022/020789
(87) International publication number: WO 2022/212080

(56) References cited:
- WO-A1-2010/078025
- WO-A1-2016/183351
- US-A1- 2013 136 698
- US-A1- 2019 125 901
- US-A1- 2019 154 697
- RAGHAVAN RAJAGOPALAN ET AL: "Hydrophilic Pyrazine Dyes as Exogenous Fluorescent Tracer Agents for Real-Time Point-of-Care Measurement of Glomerular Filtration Rate", JOURNAL OF MEDICINAL CHEMISTRY, vol. 54, no. 14, 13 June 2011 (2011-06-13), US, pages 5048 - 5058, XP055524837, ISSN: 0022-2623, DOI: 10.1021/jm200257k
- DATABASE PubChem compound ANONYMOUS : "L-Homoarginine", XP055976154, retrieved from NCBI Database accession no. 9085

## Description

### FIELD OF THE INVENTION

The present disclosure generally relates to methods of purification of the compound of Formula (I) from the precipitated reaction product comprising the compound of Formula (I), impurities, and/or residual or entrained solvents.

### BACKGROUND OF THE INVENTION

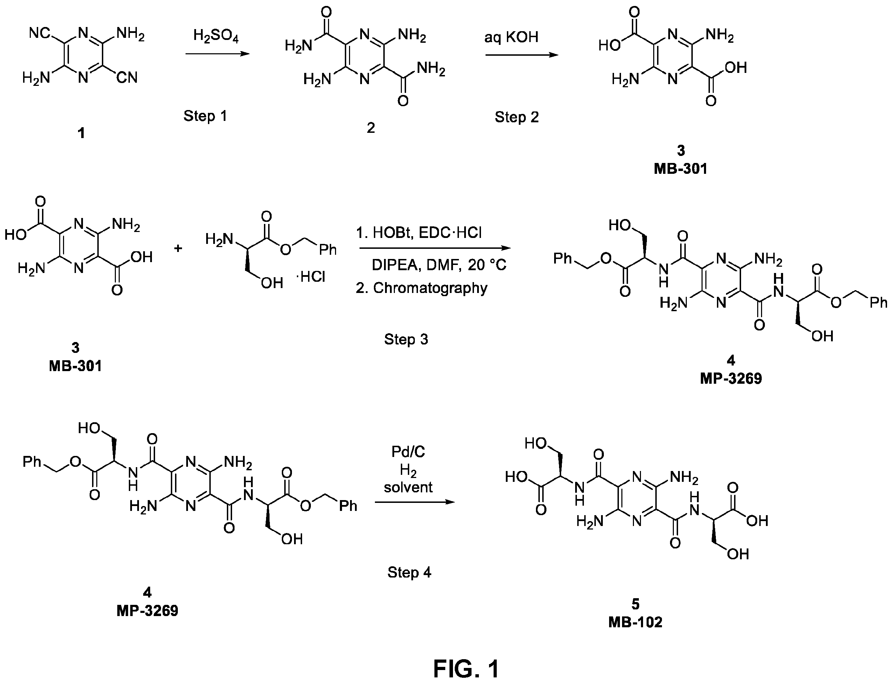

3,6-Diamino-2,5-bis{N-[(1R)-1-carboxy-2-hydroxyethyl]-carbamoyl}pyrazine] (referred to MB-102) is a non-radioactive, exogenous agents useful for the monitoring of renal function in a human subject. The preparation of 3,6-diamino-2,5-bis{N-[(1R)-1-carboxy-2-hydroxyethyl]-carbamoyl}pyrazine] is prepared through a four-step method as disclosed in the literature as shown in **FIG 1**. Step 1 of this four-step process hydrolyzes the aromatic nitrile groups of compound **1** using concentrated sulfuric acid forming the dicarboxamide compound **2.** Step 2 hydrolyzes the dicarboxamide groups on compound **2** yielding dicarboxylic acid compound **3** (referred to as MB-301). Step 3 entails the coupling of compound **3** using HOBt (hydroxybenzotriazole), EDC HCl, diisopropylethylamine in N,N-dimethylformamide with the benzyl ester of (R) serine HCl yielding the product, compound **4** (referred to MP-3269). In order to remove impurities and provide a high purity of compound **4**, the crude compound **4** is generally purified by chromatography. The purified compound **4** undergoes reductive debenzylation to form 3,6-diamino-2,5-bis{N-[(1R)-1-carboxy-2-hydroxyethyl]-carbamoyl}pyrazine] compound **5** in Step 4.

A main problem in further developing this process to a GMP (Good Manufacturing Practice) process is identified in Step 3 of the process. Step 3 utilizes chromatography and requires large quantities of various solvents to remove impurities and increase the purity of compound 4. The chromatography and the amounts of solvents increase the overall cost of the compound **4** and increases the waste for the process.

In early process development, Step 3 of the above process was examined. Step 3 was initially examined to reduce the amount of DMF in the process. This reduction in DMF would improve waste factor and reduce the cost of the process. From these initial process development experiments, the minimum amount of DMF was found necessary to provide solubility and allow Step 3 to proceed to completion. Then, DMF from the reaction mixture needed to be removed. Generally, a large excess of DMF can be removed using a large excess of water (~50-100X of water to the amount of DMF) and compound **4** would precipitate. With using large amounts of water in the precipitation, the overall waste factor of the process would increase. An alternate method to remove DMF was investigated. Experiments were conducted to remove the DMF from the completed method step through distillation before precipitation. This distillation provided a new impurity, namely the N-formyl impurity compound **12.** Attempts to use the crude compound **4** with small amounts of the N-formyl impurity **12** in Step 4 of the process yielded material containing small amounts of the N-formyl adduct which carried through from Step 3.

A new method for preparing compound **4** which utilizes Oxyma and EDC HCl as the coupling reagents is disclosed in a co-pending application (U.S. Provisional Application No. 63/168,512 filed March 31, 2021). This method is shown in **FIG. 2** and the impurities from the crude process are shown in **FIG. 3**. Other impurities were found to be easily removed through crystallization of compound **4** from Step 3.

Raghavan Rajagopalan et al. Journal of Medicinal Chemistry, 2011, vol. 54, pages 5048-5058 discloses the preparation of hydrophilic pyrazine-bis(carboxamides) derived from 3,5-diamino-pyrazine-2,5-dicarboxylic acid bearing neutral and anionic groups.

WO 2010/078025 A1 discloses compounds, preparations and formulations comprising pyrazine derivatives having multiple poly(ethylene glycol) containing substituents.

WO 2016/183351 A1 discloses compositions comprising a compound of the formula:

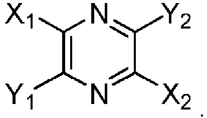

What is needed is an improved method to work-up and crystallize the impure MB-102 to form highly purified MB-102. This new method would eliminate the need for chromatography and reduce the impurities such as the N-formyl impurity compound **12.**

### FIGURES

**FIG 1** illustrates the reaction scheme to prepare 3,6-diamino-2,5-bis{N-[(1R)-1-carboxy-2-hydroxyethyl]-carbamoyl}pyrazine] as depicted in literature methods.
**FIG 2** illustrates reactions and optimization of Step 3 of the process.
**FIG 3** illustrates the impurities derived during process development.

### SUMMARY OF THE INVENTION

In one aspect, provided herein are method for purifying a compound of Formula (I) or salt thereof from the precipitated reaction product comprising the compound of Formula (I), impurities, and/or residual or entrained solvents, 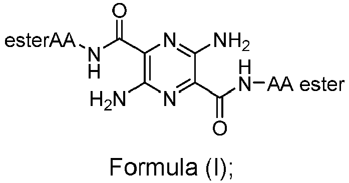 the method comprising: (a) contacting the precipitated reaction product comprising the compound of Formula (I), impurities, and/or residual or entrained solvents with ethyl acetate, iso-propanol, and water forming a first reaction mixture; (b) performing at least one distillation of the first reaction mixture under vacuum maintaining a temperature below 40°C forming a second reaction mixture; and (c) cooling the second reaction mixture to a temperature of about 0°C; and (d) isolating the compound of Formula (I) from the second reaction mixture via centrifugation or filtration; wherein the group esterAA or AAester is an ester of an amino acid wherein the ester portion is a C₁-C₁₀ unsubstituted alkyl or a C₁-C₁₀ substituted alkyl.

Other features and iterations of the invention are described in more detail below.

### DETAILED DESCRIPTION OF THE INVENTION

Before the present invention is disclosed and described, it is to be understood that this invention is not limited to the particular methods, compositions, or materials disclosed herein, but is defined by the scope of the accompanying claims.

Concentrations, amounts, and other numerical data may be expressed or presented herein in a range format. It is to be understood that such a range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 2 to about 50" should be interpreted to include not only the explicitly recited values of 2 to 50, but also include all individual values and sub-ranges within the indicated range. Thus, included in this numerical range are individual values such as 2, 2.4, 3, 3.7, 4, 5.5, 10, 10.1, 14, 15, 15.98, 20, 20.13, 23, 25.06, 30, 35.1, 38.0, 40, 44, 44.6, 45, 48, and sub-ranges such as from 1-3, from 2-4, from 5-10, from 5-20, from 5-25, from 5-30, from 5-35, from 5-40, from 5-50, from 2-10, from 2-20, from 2-30, from 2-40, from 2-50, etc. This same principle applies to ranges reciting only one numerical value as a minimum or a maximum. Furthermore, such an interpretation should apply regardless of the breadth of the range or the characteristics being described.

Provided herein are methods for the purification of the compound of Formula (I) from the precipitated reaction product comprising the compound of Formula (I), impurities, and/or residual or entrained solvents. Advantageously, the compound of Formula (I), after purification, has a lower amounts of impurities, low residual solvents, low residual water, and can be readily utilized in the subsequent process steps.

### (I) Method of Purification of the Compound of Formula (I)

The present disclosure encompasses methods for the purification of the compound of Formula (I) from the precipitated reaction product comprising the compound of Formula (I), impurities, and/or residual or entrained solvents, 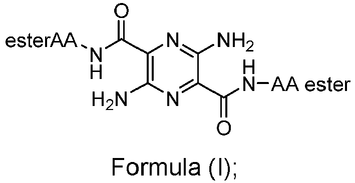 the methods comprise (a) contacting the precipitated reaction product comprising the compound of Formula (I), impurities, and/or residual or entrained solvents with ethyl acetate, iso-propanol, and water forming a first reaction mixture; (b) performing at least one distillation of the first reaction mixture under vacuum maintaining a temperature below 40°C forming a second reaction mixture; and (c) cooling the second reaction mixture to a temperature of about 0°C; and (d) isolating the compound of Formula (I) from the second reaction mixture via centrifugation or filtration; wherein the group esterAA or AAester is an ester of an amino acid wherein the ester portion is a C₁-C₁₀ unsubstituted alkyl or a C₁-C₁₀ substituted alkyl. The purification, as disclosed herein, does not utilize chromatography and reduces impurities that would carry to the next process steps or steps. Thus, these impurities would not be present or at a low level in the final product. The four-step method may be conducted in a semi-batch or batch process using normal, conventional manufacturing equipment, conventional manufacturing distillation equipment, vacuum, and conventional methods of stirring.

### (a) the compound of Formula (I)

The compound of Formula (I) is described in detail above.

Generally, the amino acid portion of the AA ester or salt thereof of the compound of Formula (I) may be a natural amino acid, an unnatural amino acid, or a synthetic amino acid. In certain embodiments, the amino acid portion of the group esterAA or AAester of the compound of Formula (I) may be an unnatural amino acid. In specific embodiments, the amino acid portion of the group esterAA or AAester of the compound of Formula (I) may be serine.

In general, the configuration of the amino acid portion of the group esterAA or AAester may be a (R) configuration, a (S) configuration, or a racemic mixture. In specific embodiments, the configuration of the amino acid portion may be an (R) configuration, specifically (R)-serine.

Generally, the ester portion of the group esterAA or AAester of the compound of Formula (I) may be a C₁-C₁₀ unsubstituted alkyl or a C₁-C₁₀ substituted alkyl. In various embodiments, the ester portion of the group esterAA or AAester of the compound of Formula (I) may be a C₁-C₈ unsubstituted alkyl or a C₁-C₈ substituted alkyl. In certain embodiments, the ester portion of the group esterAA or AAester of the compound of Formula (I) may be methyl, ethyl, tert-butyl, or benzyl. In specific embodiments, the ester portion of the group esterAA or AAester of the compound of Formula (I) is benzyl.

The reaction product from the process comprises the compound of Formula (I), increased impurities, and/or residual or entrained solvents.

### a. step (a)

As discussed above, Step (a) of the four-step process involves contacting the precipitated reaction product comprising the compound of Formula (I), impurities, and/or residual or entrained solvents with ethyl acetate, iso-propanol, and water forming a first reaction mixture.

The solvents used in the methodprovide a partial or total solubility of the precipitated reaction product from the process comprising the compound of Formula (I), impurities and/or residual or entrained solvents.

The water used in step (a) of the four-step method may be deionized water, distilled water, distilled deionized water, or potable (tap) water.

In general, the solvent to the compound of Formula (I) are at a volume (mL) to weight ratio (g) from 2:1 to 25:1. In various embodiments, the volume to weight ratio may be 2:1 to 25:1, from 4:1 to 20:1, or from 5:1 to 18.5:1. In one embodiment, the solvent to the compound of Formula (I) are at a volume (mL) to weight ratio (g) from 15:1 to 20:1. In another embodiment, the solvent to the compound of Formula (I) are at a volume (mL) to weight ratio (g) from 4:1 to 6:1.

In general, Step (a) of the four-step method may be conducted at a temperature that ranges from 10°C to 35°C depending on the solvent or mixtures of solvents utilized. In various embodiments, the temperature of the step (a) may range from 10°C to 35°C, from 15°C to 30°C, or from 20°C to 30°C. In one embodiment, the step (a) of the method may be conducted at temperature of 23 °C (room temperature). The method is typically performed under ambient pressure. The reaction may also be conducted under an inert atmosphere, for example under nitrogen, argon, helium, and combinations thereof and air.

Generally, Step (a) of the four-step method is allowed to proceed for a sufficient period of time until a slurry is obtained. The duration of the Step (a) of the four-step method may range from 30 minutes to 6 hours. In some embodiments, the duration of the method may range from 30 minutes to 2 hours, from 2 hours to 4 hours, or from 4 hours to 6 hours until a slurry is obtained. In one embodiment, the step (a) of the four-step method may be allowed to proceed for 30 minutes to 1 hour.

### b. step (b)

Step (b) of the four-step method comprises performing at least one distillation of the first reaction mixture under vacuum maintaining a temperature below 40°C forming a second reaction mixture. This step removes the entrained, a solvate, or residual solvent in the precipitated reaction product comprising the compound of Formula (I) which was not adequately removed during the precipitation. Step (b) of the four-step method may utilize at least two distillations wherein the second or more distillations may utilize the same solvent or a different solvent as in the first. If a second distillation is utilized, additional solvent may be added to the first reaction mixture before the second distillation. In various embodiments, the solvent is the same solvent or mixture of solvents. In other embodiments, the solvent may be a different solvent or a different mixture of solvents.

Generally, the temperature of Step (b) of the four-step method is conducted to maintain the temperature of the contents of the first reaction mixture under 40°C. In various embodiments, the contents of the first reaction mixture are maintained at a temperature under 40°C, under 30°C, under 20°C, under 10°C, or under 0°C. In one embodiment, the temperature of the contents of the first reaction mixture is maintained under 20°C.

In general, the vacuum used in the distillation in Step (b) of the four-step method is less than 1270 mm (50 inches) of Hg. In various embodiments, the vacuum in the distillation of step (b) is less than 1270 mm (50 inches) of Hg, less than 1020 mm (40 inches) of Hg, less than 760 mm (30 inches) of Hg, or less than 510 mm (20 inches) of Hg. In one embodiment, the vacuum used in the distillation of step (b) of the four-step method is less than 711 mm (28 inches) of Hg.

Generally, the vacuum distillation of Step (b) of the four-step method is conducted until the volume of the first reaction mixture is less than 50% as compared to the initial volume of the first reaction mixture. In various embodiments, the distillation of Step (b) of the four-step method is conducted until the volume is less than 50%, less than 40%, less than 30%, or less than 20% by volume as compared to the initial volume of the first reaction mixture. In one embodiment, the distillation is conducted until the volume of the first reaction mixture is less than 50% as compared to the initial volume of the first reaction mixture. In another embodiment, the distillation is conducted until the volume of the first reaction mixture is less than 25% as compared to the initial volume of the first reaction mixture. If more than one distillation is utilized in Step (b) of the four-step method, the volume of the first reaction mixture after distillation may be the same or different as compared to the initial volume of the first reaction mixture. In another embodiment, the first reaction mixture is distilled in the first distillation to a volume of less than 50% as compared to the initial volume of the first reaction mixture and distilled in the second distillation to a volume less than 25% as compared to the initial volume of the first reaction mixture.

As appreciated by the skilled artisan, the time to conduct Step (b) of the four-step method can and will vary depending on the number of distillations, the size of the batch, the volume of the solvent, the amount of vacuum, and the equipment used.

### c. step (c)

Step (c) of the four-step method cools the second reaction mixture formed after Step (b) and crystallizes the purified compound of Formula (I). The crystals may be of various sizes and shapes. The second reaction mixture may be externally cooled to further reduce the solubility of the compound of Formula (I).

Generally, the second reaction mixture is cooled to a temperature of about 0 °C..

### d. step (d)

Step (d) of the four-step method isolates the purified compound of Formula (I) from the second reaction mixture. The compound comprises purified crystals of the compound of Formula (I).

The purified compound of Formula (I) is isolated via filtration or centrifugation. After isolating the purified compound of Formula (I), this material may be washed and dried until a constant amount of moisture is obtained as measured by a Karl Fisher titration (KF).

In general, the yield of the compound of Formula (I) after conducting the four-step method may be greater than 60%. In various embodiments, the yield may be greater than 60%, greater than 70%, greater than 80%, or greater than 90%. In one embodiment, the yield of the four-step method is greater than 70%.

Generally, the purity of the compound of Formula (I) may be greater than 95% as measured by high-performance liquid chromatography (HPLC), ultra-performance liquid chromatography (UPLC), or other methods known in the art. In various embodiments, the purity of the compound of Formula (I) may be greater than 95%, greater than 97%, greater than 99%, or greater than 99.9%. In one embodiment, the purity of the compound of Formula (I) is greater than 97%.

### d. exemplary embodiments

In some embodiments, the ester portion of the group esterAA or AAester of the compound of Formula (I) is (R)-benzyl serine; the solvent is a mixture of ethyl acetate, iso-propanol, and water in Step (a); the solvent to the compound of Formula (I) has a volume (mL) to weight ratio (g) ratio from 2:1 to 25:1; the first reaction mixture is distilled under vacuum; the temperature of the distillation is less than 40°C the vacuum is less than 1270 mm (50 inches) of Hg; the volume of the first reaction mixture after distillation is less than 50%; the solvent is isopropanol in Step (b); the solvent to the compound of Formula (I) has a volume (mL) to weight ratio (g) ratio from 2:1 to 25:1; the first reaction mixture is distilled under vacuum; the vacuum is less than 1270 mm (50 inches) of Hg; the temperature of the distillation is less than 20°C; the volume of the first reaction mixture after distillation is less than 50% forming a second reaction mixture; the second reaction mixture is cooled to a temperature of about 0°C; and the compound of Formula (I) is isolated from the second reaction mixture. The yield after conducting the four-step process of the compound of Formula (I) is greater than 60% and the purity of the compound of Formula (I) is greater than 95%.

In certain embodiments, the ester portion of the group esterAA or AAester of the compound of Formula (I) is (R)-benzyl serine; the solvent is a mixture of ethyl acetate, iso-propanol, and water in Step (a); the solvent to the compound of Formula (I) has a volume (mL) to weight ratio (g) ratio from 15:1 to 20:1; the first reaction mixture is distilled under vacuum; the temperature of the distillation is less than 40°C, the vacuum is less than 1270 mm (50 inches) of Hg; the volume of the first reaction mixture after distillation is less than 50%; the solvent is isopropanol in Step (b); the solvent to the compound of Formula (I) has a volume (mL) to weight ratio (g) ratio from 4:1 to 6:1; the first reaction mixture is distilled under vacuum; the temperature of the distillation is less than 20°C; the vacuum is less than 635 mm (25 inches) of Hg; the volume of the first reaction mixture after distillation is less than 25% forming a second reaction mixture; and the second reaction mixture is cooled to a temperature of about 0°C; and the compound of Formula (I) is isolated from the second reaction mixture. The yield after conducting the four-step process of the compound of Formula (I) is greater than 70% and the purity of the compound of Formula (I) is greater than 97%.

### DEFINITIONS

When introducing elements of the embodiments described herein, the articles "a", "an", "the" and "said" are intended to mean that there are one or more of the elements. The term "about" is intended to represent ± 20%.

In this disclosure, "comprises," "comprising," "containing," and "having" and the like can have the meaning ascribed to them in U.S. Patent Law and can mean "includes," "including," and the like, and are generally interpreted to be open ended terms. The terms "consisting of" or "consists of" are closed terms, and include only the components, structures, steps, or the like specifically listed in conjunction with such terms, as well as that which is in accordance with U.S. Patent law. "Consisting essentially of" or "consists essentially of" have the meaning generally ascribed to them by U.S. Patent law. In particular, such terms are generally closed terms, with the exception of allowing inclusion of additional items, materials, components, steps, or elements, that do not materially affect the basic and novel characteristics or function of the item(s) used in connection therewith. For example, trace elements present in a composition, but not affecting the composition's nature or characteristics would be permissible if present under the "consisting essentially of" language, even though not expressly recited in a list of items following such terminology. In this specification when using an open ended term, like "comprising" or "including," it is understood that direct support should be afforded also to "consisting essentially of" language as well as "consisting of" language as if stated explicitly and vice versa.

As various changes could be made in the above-described methods without departing from the scope of the invention, it is intended that all matter contained in the above description and in the examples given below, shall be interpreted as illustrative and not in a limiting sense.

### EXAMPLES

### Example 1: Purification of Dibenzyl 2,2'-((3,6-diaminopyrazine-2,5-dicarbonyl)bis(azanediyl)(2R,2'R)-bis(3-hydroxypropanoate) (Reference Example)

59.02 g of impure dibenzyl 2,2'-((3,6-diaminopyrazine-2,5-dicarbonyl)bis(azanediyl)(2R,2'R)-bis(3-hydroxypropanoate) was reconstituted in EtOAc (500 mL, 8.5 vol) and generated an orange slurry. IPA (295 mL, 5 vol) was added at 20 °C and some solubility was observed. H₂O (295 mL, 5 vol) was added to this mixture and a biphasic and homogenous mixture was obtained. Vacuum distillation was used to remove solvent at Tⱼ = 20 °C / Tᵣ = 0 °C (711 mm [28 in] Hg) to a volume of 600 mL. As EtOAc was removed an orange slurry was generated and the liquor became a single phase. IPA (295 mL, 5 vol) was charged and vacuum distillation (711 mm [28 in] Hg) was continued at Tⱼ = 25°C / Tᵣ = 13 °C until a Vᵣ = 425 mL (~7.7 vol cf. theoretical yield). Vacuum was broken and the orange slurry was cooled to 3 °C and aged for 1 h prior to cold filtration. The reaction vessel was rinsed with IPA/H₂O (1:1 v/v; 120 mL, 2.18 vol), chilled, and was then used to rinse the wet cake. The volume of filtrate obtained from the crystallization of MP-3269 was 219 mL (4 vol). The wet cake was dried in a vacuum oven at 35 °C for 16.5 h to provided 39.04 g MP-3269 (70% yield) as an orange free flowing solid. Analysis by HPLC (25 minute method) indicated a purity of 97.28% AUC and contained 1.17% impurity at RRT = 1.03 (m/z = 233, positive mode). The material contained 0.59% H₂O by KF analysis.

## Claims

1. A method for purifying a compound of Formula (I) or salt thereof from a precipitated reaction product comprising the compound of Formula (I), impurities, and/or residual or entrained solvents, 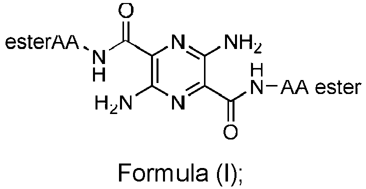 the method comprising:
(a) contacting the precipitated reaction product comprising the compound of Formula (I), impurities, and/or residual or entrained solvents with ethyl acetate, iso-propanol, and water forming a first reaction mixture;
(b) performing at least one distillation of the first reaction mixture under vacuum maintaining a temperature below 40°C forming a second reaction mixture; and
(c) cooling the second reaction mixture to a temperature of about 0°C; and
(d) isolating the compound of Formula (I) from the second reaction mixture via centrifugation or filtration;
wherein the group esterAA or AAester is an ester of an amino acid wherein the ester portion is a C₁-C₁₀ unsubstituted alkyl or a C₁-C₁₀ substituted alkyl.

2. The method of claim 1, wherein the amino acid portion of the group esterAA or AAester of the compound of Formula (I) is a natural amino acid, an unnatural amino acid, or a synthetic amino acid, and/or wherein the amino acid portion of the group esterAA or AAester of the compound of Formula (I) is a (R) configuration, a (S) configuration, or a racemic mixture, such as wherein the amino acid portion of the group esterAA or AAester of the compound of Formula (I) is (R)-serine.

3. The method of any of claims 1-2, wherein the ester portion of the group esterAA or AAester of the compound of Formula (I) is a C₁-C₈ unsubstituted alkyl or a C₁-C₈ substituted alkyl; or wherein the ester portion of the group esterAA or AAester of the compound of Formula (I) is methyl, ethyl, *tert-*butyl, or benzyl, such as wherein the ester portion of the group esterAA or AAester of the compound of Formula (I) is benzyl.

4. The method of any of claims 1-3, wherein the at least one distillation in step (b) utilizes at least two distillations; and/or wherein the first reaction mixture is distilled to a volume of less than 50% of the initial volume of the first reaction mixture, such as wherein the first reaction mixture is distilled to a volume less than 25% of the initial volume of the first reaction mixture.

5. The method of claim 4, wherein additional solvent is added between distillations, wherein the additional solvent may be the same or different to that used in the first distillation.

6. The method of any of claims 1-5, wherein the solvent to the compound of Formula (I) are at a volume (mL) to weight ratio (g) from 2:1 to 25:1, such as wherein the solvent to the compound of Formula (I) are at a volume (mL) to weight ratio (g) from 15:1 to 20:1, or such as wherein the solvent to the compound of Formula (I) are at a volume (mL) to weight ratio (g) from 4:1 to 6:1.

7. The method of any of claims 1-6, wherein the temperature is maintained in step (b) to less than 20°C.

8. The method of any of claims 1-7, wherein the vacuum is conducted at less than 1270 mm (50 inches) of Hg, such as wherein the vacuum is less than 711 mm (28 inches) of Hg.

9. The method of claim 1, wherein the ester portion of the AA ester or salt thereof of the compound of Formula (I) is (R)-benzyl serine; the solvent to the compound of Formula (I) has a volume (mL) to weight ratio (g) ratio from 2:1 to 25:1; the first reaction mixture is distilled under vacuum; the temperature of the distillation is less than 40°C, the vacuum is less than 1270 mm (50 inches) of Hg; the volume of the first reaction mixture after distillation is less than 50%; the solvent is isopropanol in Step (b); the solvent to the compound of Formula (I) has a volume (mL) to weight ratio (g) ratio from 2:1 to 25:1; the first reaction mixture is distilled under vacuum; the vacuum is less than 1270 mm (50 inches) of Hg; the temperature of the distillation is less than 20°C; the volume of the first reaction mixture after distillation is less than 50% forming a second reaction mixture; the second reaction mixture is cooled to a temperature of about 0°C; and the compound of Formula (I) is isolated from the second reaction mixture.

10. The method of any of claims 1-9, wherein the ester portion of the AA ester or salt thereof of the compound of Formula (I) is (R)-benzyl serine; the solvent to the compound of Formula (I) has a volume (mL) to weight ratio (g) ratio from 15:1 to 20:1; the first reaction mixture is distilled under vacuum; the temperature of the distillation is less than 40°C, the vacuum is less than 1270mm (50 inches) of Hg; the volume of the first reaction mixture after distillation is less than 50%; the solvent is isopropanol in Step (b); the solvent to the compound of Formula (I) has a volume (mL) to weight ratio (g) ratio from 4:1 to 6:1; the first reaction mixture is distilled under vacuum; the temperature of the distillation is less than 20°C; the vacuum is less than 635mm (25 inches) of Hg; the volume of the first reaction mixture after distillation is less than 25% forming a second reaction mixture; the second reaction mixture is cooled to a temperature of about 0°C; and the compound of Formula (I) is isolated from the second reaction mixture.

## Patentansprüche

1. Verfahren zur Reinigung einer Verbindung der Formel (I) oder eines Salzes davon aus einem ausgefällten Reaktionsprodukt, das die Verbindung der Formel (I), Verunreinigungen und/oder restliche oder mitgerissene Lösungsmittel umfasst, 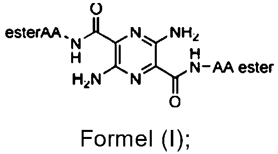 wobei das Verfahren Folgendes umfasst:
(a) Kontaktieren des ausgefällten Reaktionsprodukts, das die Verbindung der Formel (I), Verunreinigungen und/oder restliche oder mitgerissene Lösungsmittel umfasst, mit Ethylacetat, Iso-propanol und Wasser unter Bildung eines ersten Reaktionsgemisches;
(b) Durchführen mindestens einer Destillation des ersten Reaktionsgemisches unter Vakuum, wobei eine Temperatur unter 40 °C aufrechterhalten wird, unter Bildung eines zweiten Reaktionsgemisches; und
(c) Abkühlen des zweiten Reaktionsgemisches auf eine Temperatur von etwa 0 °C; und
(d) Isolieren der Verbindung der Formel (I) aus dem zweiten Reaktionsgemisch durch Zentrifugation oder Filtration;
wobei die Gruppe esterAA oder AAester ein Ester einer Aminosäure ist, wobei der Esteranteil ein unsubstituiertes C₁-C₁₀-Alkyl oder ein substituiertes C₁-C₁₀-Alkyl ist.

2. Verfahren nach Anspruch 1, wobei der Aminosäureanteil der Gruppe esterAA oder AAester der Verbindung der Formel (I) eine natürliche Aminosäure, eine unnatürliche Aminosäure oder eine synthetische Aminosäure ist, und/oder wobei der Aminosäureanteil der Gruppe esterAA oder AAester der Verbindung der Formel (I) eine (R)-Konfiguration, eine (S)-Konfiguration oder ein racemisches Gemisch ist, wie etwa wobei der Aminosäureanteil der Gruppe esterAA oder AAester der Verbindung der Formel (I) (R)-Serin ist.

3. Verfahren nach einem der Ansprüche 1-2, wobei der Esteranteil der Gruppe esterAA oder AAester der Verbindung der Formel (I) ein unsubstituiertes C₁-C₈-Alkyl oder ein substituiertes C₁-C₈-Alkyl ist; oder wobei der Esteranteil der Gruppe esterAA oder AAester der Verbindung der Formel (I) Methyl, Ethyl, tert-Butyl oder Benzyl ist, wie etwa wobei der Esteranteil der Gruppe esterAA oder AAester der Verbindung der Formel (I) Benzyl ist.

4. Verfahren nach einem der Ansprüche 1-3, wobei die mindestens eine Destillation in Schritt (b) mindestens zwei Destillationen verwendet; und/oder wobei das erste Reaktionsgemisch auf ein Volumen von weniger als 50 % des Anfangsvolumens des ersten Reaktionsgemisches destilliert wird, wie etwa wobei das erste Reaktionsgemisch auf ein Volumen von weniger als 25 % des Anfangsvolumens des ersten Reaktionsgemisches destilliert wird.

5. Verfahren nach Anspruch 4, wobei zwischen den Destillationen zusätzliches Lösungsmittel zugegeben wird, wobei das zusätzliche Lösungsmittel das gleiche wie das in der ersten Destillation verwendete oder ein davon verschiedenes sein kann.

6. Verfahren nach einem der Ansprüche 1-5, wobei das Lösungsmittel und die Verbindung der Formel (I) in einem Volumen-(mL)-zu-Gewichts-(g)-Verhältnis von 2:1 bis 25:1 vorliegen, wie etwa wobei das Lösungsmittel und die Verbindung der Formel (I) in einem Volumen-(mL)-zu-Gewichts-(g)-Verhältnis von 15:1 bis 20:1 vorliegen, oder wie etwa wobei das Lösungsmittel und die Verbindung der Formel (I) in einem Volumen-(mL)-zu-Gewichts-(g)-Verhältnis von 4:1 bis 6:1 vorliegen.

7. Verfahren nach einem der Ansprüche 1-6, wobei die Temperatur in Schritt (b) auf weniger als 20 °C gehalten wird.

8. Verfahren nach einem der Ansprüche 1-7, wobei das Vakuum bei weniger als 1270 mm (50 Zoll) Hg durchgeführt wird, wie etwa wobei das Vakuum weniger als 711 mm (28 Zoll) Hg beträgt.

9. Verfahren nach Anspruch 1, wobei der Esteranteil des AA-Esters oder Salzes davon der Verbindung der Formel (I) (R)-Benzylserin ist; das Lösungsmittel zu der Verbindung der Formel (I) ein Volumen-(mL)-zu-Gewichts-(g)-Verhältnis von 2:1 bis 25:1 aufweist; das erste Reaktionsgemisch unter Vakuum destilliert wird; die Temperatur der Destillation weniger als 40 °C beträgt, das Vakuum weniger als 1270 mm (50 Zoll) Hg beträgt; das Volumen des ersten Reaktionsgemisches nach der Destillation weniger als 50 % beträgt; das Lösungsmittel in Schritt (b) Isopropanol ist; das Lösungsmittel zu der Verbindung der Formel (I) ein Volumen-(mL)-zu-Gewichts-(g)-Verhältnis von 2:1 bis 25:1 aufweist; das erste Reaktionsgemisch unter Vakuum destilliert wird; das Vakuum weniger als 1270 mm (50 Zoll) Hg beträgt; die Temperatur der Destillation weniger als 20 °C beträgt; das Volumen des ersten Reaktionsgemisches nach der Destillation weniger als 50 % beträgt, unter Bildung eines zweiten Reaktionsgemisches; das zweite Reaktionsgemisch auf eine Temperatur von etwa 0 °C abgekühlt wird; und die Verbindung der Formel (I) aus dem zweiten Reaktionsgemisch isoliert wird.

10. Verfahren nach einem der Ansprüche 1-9, wobei der Esteranteil des AA-Esters oder Salzes davon der Verbindung der Formel (I) (R)-Benzylserin ist; das Lösungsmittel zu der Verbindung der Formel (I) ein Volumen-(mL)-zu-Gewichts-(g)-Verhältnis von 15:1 bis 20:1 aufweist; das erste Reaktionsgemisch unter Vakuum destilliert wird; die Temperatur der Destillation weniger als 40 °C beträgt, das Vakuum weniger als 1270 mm (50 Zoll) Hg beträgt; das Volumen des ersten Reaktionsgemisches nach der Destillation weniger als 50 % beträgt; das Lösungsmittel in Schritt (b) Isopropanol ist; das Lösungsmittel zu der Verbindung der Formel (I) ein Volumen-(mL)-zu-Gewichts-(g)-Verhältnis von 4:1 bis 6:1 aufweist; das erste Reaktionsgemisch unter Vakuum destilliert wird; die Temperatur der Destillation weniger als 20 °C beträgt; das Vakuum weniger als 635 mm (25 Zoll) Hg beträgt; das Volumen des ersten Reaktionsgemisches nach der Destillation weniger als 25 % beträgt, unter Bildung eines zweiten Reaktionsgemisches; das zweite Reaktionsgemisch auf eine Temperatur von etwa 0 °C abgekühlt wird; und die Verbindung der Formel (I) aus dem zweiten Reaktionsgemisch isoliert wird.

## Revendications

1. Procédé pour purifier un composé de formule (I) ou son sel à partir d'un produit réactionnel précipité comprenant le composé de formule (I), des impuretés et/ou des solvants résiduels ou entrainés, 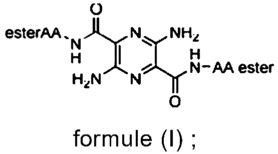 le procédé comprenant :
(a) la mise en contact du produit de réaction précipité comprenant le composé de formule (I), des impuretés et/ou des solvants résiduels ou entraînés avec de l'acétate d'éthyle, de l'isopropanol et de l'eau, formant un premier mélange réactionnel ;
(b) la réalisation d'au moins une distillation du premier mélange réactionnel sous vide en maintenant une température inférieure à 40 °C, formant un deuxième mélange réactionnel ; et
(c) le refroidissement du deuxième mélange réactionnel à une température d'environ 0 °C ; et
(d) l'isolement du composé de formule (I) à partir du deuxième mélange réactionnel par centrifugation ou filtration ;
dans lequel le groupe esterAA ou AAester est un ester d'un acide aminé dans lequel la portion ester est un alkyle non substitué en C₁-C₁₀ ou un alkyle substitué en C₁-C₁₀.

2. Procédé selon la revendication 1, dans lequel la portion d'acide aminé du groupe esterAA ou AAester du composé de formule (I) est un acide aminé naturel, un acide aminé non naturel ou un acide aminé synthétique, et/ou dans lequel la portion d'acide aminé du groupe esterAA ou AAester du composé de formule (I) est une configuration (R), une configuration (S) ou un mélange racémique, tel que dans lequel la portion d'acide aminé du groupe esterAA ou AAester du composé de formule (I) est la (R)-sérine.

3. Procédé selon l'une quelconque des revendications 1 à 2, dans lequel la portion ester du groupe esterAA ou AAester du composé de formule (I) est un alkyle non substitué en C₁-C₈ ou un alkyle substitué en C₁-C₈ ; ou dans lequel la portion ester du groupe esterAA ou AAester du composé de formule (I) est du méthyle, de l'éthyle, du tert-butyle ou du benzyle, tel que dans lequel la portion ester du groupe esterAA ou AAester du composé de formule (I) est le benzyle.

4. Procédé selon l'une quelconque des revendications 1 à 3, dans lequel l'au moins une distillation à l'étape (b) utilise au moins deux distillations ; et/ou dans lequel le premier mélange réactionnel est distillé à un volume inférieur à 50 % du volume initial du premier mélange réactionnel, tel que dans lequel le premier mélange réactionnel est distillé à un volume inférieur à 25 % du volume initial du premier mélange réactionnel.

5. Procédé selon la revendication 4, dans lequel un solvant supplémentaire est ajouté entre les distillations, dans lequel le solvant supplémentaire peut être identique ou différent de celui utilisé dans la première distillation.

6. Procédé selon l'une quelconque des revendications 1 à 5, dans lequel le solvant au composé de formule (I) sont à un rapport volume (ml)/poids (g) de 2:1 à 25:1, tel que dans lequel le solvant au composé de formule (I) sont à un rapport volume (ml)/poids (g) de 15:1 à 20:1, ou tel que dans lequel le solvant au composé de formule (I) sont à un rapport volume (ml)/poids (g) de 4:1 à 6:1.

7. Procédé selon l'une quelconque des revendications 1 à 6, dans lequel la température est maintenue à l'étape (b) à une température inférieure à 20 °C.

8. Procédé selon l'une quelconque des revendications 1 à 7, dans lequel le vide est conduit à moins de 1270 mm (50 pouces) de Hg, tel que dans lequel le vide est inférieur à 711 mm (28 pouces) de Hg.

9. Procédé selon la revendication 1, dans lequel la portion ester de l'ester AA ou son sel du composé de formule (I) est la (R)-benzylsérine ; le solvant au composé de formule (I) présente un rapport volume (ml)/poids (g) de 2:1 à 25:1 ; le premier mélange réactionnel est distillé sous vide ; la température de la distillation est inférieure à 40 °C, le vide est inférieur à 1270 mm (50 pouces) de Hg ; le volume du premier mélange réactionnel après distillation est inférieur à 50 % ; le solvant est de l'isopropanol à l'étape (b) ; le solvant au composé de formule (I) présente un rapport volume (ml)/poids (g) de 2:1 à 25:1 ; le premier mélange réactionnel est distillé sous vide ;
le vide est inférieur à 1270 mm (50 pouces) de Hg ; la température de la distillation est inférieure à 20 °C ; le volume du premier mélange réactionnel après distillation est inférieur à 50 %, formant un deuxième mélange réactionnel ; le deuxième mélange réactionnel est refroidi à une température d'environ 0 °C ; et le composé de formule (I) est isolé du deuxième mélange réactionnel.

10. Procédé selon l'une quelconque des revendications 1 à 9, dans lequel la portion ester de l'ester AA ou son sel du composé de formule (I) est la (R)-benzylsérine ; le solvant du composé de formule (I) présente un rapport volume (ml)/poids (g) de 15:1 à 20:1 ; le premier mélange réactionnel est distillé sous vide ; la température de la distillation est inférieure à 40 °C, le vide est inférieur à 1270 mm (50 pouces) de Hg ; le volume du premier mélange réactionnel après distillation est inférieur à 50 % ; le solvant est de l'isopropanol à l'étape (b) ; le solvant au composé de formule (I) présente un rapport volume (ml)/poids (g) de 4:1 à 6:1 ; le premier mélange réactionnel est distillé sous vide ; la température de la distillation est inférieure à 20 °C ; le vide est inférieur à 635 mm (25 pouces) de Hg ;
le volume du premier mélange réactionnel après distillation est inférieur à 25 %, formant un deuxième mélange réactionnel ; le deuxième mélange réactionnel est refroidi à une température d'environ 0 °C ; et le composé de formule (I) est isolé du deuxième mélange réactionnel.
